# EUROPÄISCHE PATENTANMELDUNG

(11) **EP 1 746 087 A1**
(43) Veröffentlichungstag der Anmeldung: **24.01.2007**
(21) Anmeldenummer: 05015916.9
(22) Anmeldetag: 21.07.2005
(51) Int. Cl.: C07D 209/12, C07D 209/14, C07D 209/18, C07D 401/12, C07D 403/12, A61K 31/4439, A61K 31/404, A61P 35/00, A61P 31/00

(54) **3-Indolylmethylen-Derivate mit cytostatischer Wirkung**

(71) Anmelder: Universitaet Regensburg, 93053 Regensburg (DE)
(72) Erfinder: Von Angerer, Erwin, D-93077 Bad Abbach (DE)
(74) Vertreter: Behnisch, Werner

(57) **Zusammenfassung**

Die Erfindung betrifft 3-Indolylmethylen-Derivate mit der allgemeinen Formel **II** mit cytostatischer Wirkung, sowie ein Verfahren zur Herstellung der 3-Indolylmethylen-Derivate, deren Verwendung zur Herstellung von Arzneimitteln sowie 3-Indolylmethylen-Derivate enthaltende pharmazeutische Zusammensetzungen.

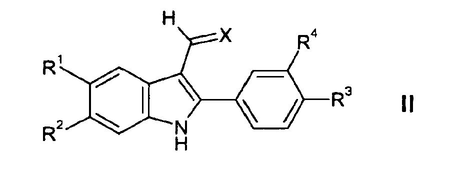

## Beschreibung

Die vorliegende Erfindung betrifft neuartige 3-Indolylmethylen-Derivate, ein Verfahren zur Herstellung von 3-Indolylmethylen-Derivaten, deren Verwendung zur Herstellung von Arzneimitteln, sowie 3-Indolylmethylen-Derivate enthaltende pharmazeutische Präparate.

Das Wachstum maligner Gewebe ist durch erhöhte Zellproliferation, unkontrollierte Zellvermehrung und eingeschränkte apoptotische Regulation gekennzeichnet. Eingriffe in diese Prozesse haben sich in der Behandlung von Tumorerkrankungen bewährt. Als besonders geeignet haben sich Substanzen erwiesen, die gezielt in den Zellzyklus eingreifen.

In der Literatur sind bereits verschiedene Verbindungen beschrieben, die die Ausbildung funktioneller Mikrotubuli, die u.a. für die Zellteilung benötigt werden, hemmen (Curr.Opin.Drug Discovery Dev. 2000, 3, 575). Dies kann über eine Hemmung der Tubulinpolymerisation geschehen, wie es beim Vincristin der Fall ist (Exp.Opin.Ther.Patents 1999, 9, 1069), oder durch Stabilisierung der Mikrotubuli, wie sie etwa durch Paclitaxel hervorgerufen wird. Beide Substanzen sind Naturstoffe und zeichnen sich durch äußerst komplexe chemische Strukturen aus.

Strukturell einfachere Verbindungen, die die Tubulinpolymerisation hemmen, leiten sich vom cis-Stilbenderivat Combretastatin A4 ab (US 5,561,122; US 5,731,353; US 5,674,906; JP 8,087,656). Auch verschiedene heterozyklische Systeme sind in der Patentliteratur beschrieben, beispielsweise Chinolone (WO96/10563) und Naphthiridinone (WO98/39332). In WO98/39323 wird ein 2-Phenylbenzothiophen-Derivat mit einem substituierten Benzolylrest in 3-Stellung als Hemmstoff der Tubulinpolymerisation beschrieben, dessen Wirkung aber nur unvollständig ist (Exp.Opin.Ther.Patents 1999, 9, 1069).

Ein neuer Ansatzpunkt, um die Zellvermehrung zu unterdrücken, war die Hemmung von Zellzyklus-abhängigen Kinasen (CDK), insbesondere CDK 2 (C. Benson et al. Brit.J.Cancer 2005,92,7-12). Ein Beispiel dafür ist Flavopiridol, das den Zellzyklus in der G₁-Phase blockiert und sich gegenwärtig in der klinischen Entwicklung befindet. Ein weiterer niedermolekularer Hemmstoff, der in der G₁-Phase des Zellzyklus eingreift, ist Indisulam (E7070)( C.T. Supuran, Expert Opin. Investig. Drugs, 2003,12,283-7), das zwar auch einen Indolring besitzt, aber sonst keinerlei strukturelle Ähnlichkeiten mit den erfindungsgemäßen Verbindungen zeigt.

In der Publikation Goldbrunner et al., J.Med.Chem. 1997, 40, 3524, sind hydroxylierte Indolo[2,1-a]isochinoline als neuartige Hemmstoffe der Tubulinpolymerisation beschrieben. Methoxysubstituierte 2-Phenylindol-3-carbaldehyde, die strukturell damit verwandt sind, wirken in Zellkultur stark cytostatisch, wobei als Wirkmechanismus eine Bindung an das Tubulin und eine Hemmung der Tubulinpolymerisation nachgewiesen wurde (J.Med.Chem. 1998, 41, 4965).

Hemmstoffe der Tubulinpolymerisation führen zu einer Blockade des Zellzyklus in der G₂/M-Phase und initieren häufig eine Apoptose der Zellen ("programmierter Zelltod"). Die beiden letztgenannten zellulären Ereignisse spielen besonders bei Krebszellen eine große Rolle, da diese eine wesentlich höhere Proliferationsrate als die meisten gesunden Zellen aufweisen. Im Gegensatz zu verschiedenen Naturstoffen mit komplexer chemischer Struktur haben niedermolekulare Verbindungen, die in den Zellzyklus eingreifen, bisher keinen Eingang in die etablierte Krebschemotherapie gefunden.

Der Erfindung liegt das Problem zugrunde, niedermolekulare Wirkstoffe mit einfacher chemischer Struktur zu finden, die das Wachstum von Tumorzellen über einen Eingriff in den Zellzyklus hemmen und zur Behandlung insbesondere von Krebserkrankungen geeignet sind. Der Erfindung liegt weiterhin das Problem zugrunde, einen cytostatischen Wirkstoff bereitzustellen, der nicht am Tubulinsystem angreifen und daher die mit diesem Effekt verbundenen unerwünschten Wirkungen nicht zeigt.

Das Problem wird durch den Gegenstand der unabhängigen Ansprüche gelöst. Bevorzugte Ausführungsformen der Erfindung sind in den Unteransprüchen angegeben.

Die erfindungsgemäßen 3-Indolylmethylen-Derivate weisen das gemeinsame Strukturteil mit der allgemeinen Formel 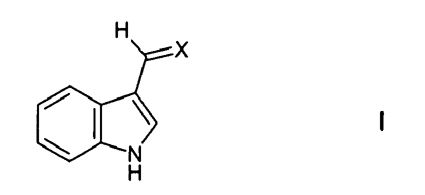 als Bestandteil von Verbindungen mit cytostatischer Wirkung auf. In dieser Formel kann
**X** folgende Bedeutungen haben:
a) eine Methylengruppe mit einer oder zwei Carboxylgruppen oder
b) eine Methylengruppe mit einer oder zwei Carbonsäureamidgruppen, wobei die Aminogruppen entweder unsubstituiert oder mit C₁-C₆-Alkylgruppen substituiert sind, oder
c) eine Methylengruppe mit einer oder zwei Nitrilgruppen oder
d) eine Methylengruppe mit zwei unterschiedlichen unter a), b) oder c) genannten Substituenten oder
e) eine Gruppierung NR⁶, wobei R⁶ Hydroxy, Alkoxy mit einem gerad- oder verzweigtkettigen C₁ ― C₆-Alkylrest, ein gerad- oder verzweigtkettiger C₁- C₆-Alkylrest, oder ein Arylrest, wobei Aryl ein unsubstituierter oder substituierter, vorzugsweise ein- oder zweifach substituierter Phenylrest ist,
f) eine Gruppierung N-NR⁷R⁸, wobei R⁷ ein Wasserstoffatom oder eine gerad- oder verzweigtkettige C₁ - C₄-Alkylgruppe bedeutet und R⁸ ein unsubstituierter oder substituierter, vorzugsweise ein- oder zweifach substituierter Phenylrest ist,
g) eine Gruppierung N-NR¹⁰-CO-R⁹, wobei R⁹ eine Aminogruppe, ein unsubstituierter oder substituierter, vorzugsweise ein- oder zweifach substituierter Phenylrest oder Heteroarylrest, beispielsweise 2-Thienyl, 2-Furyl, 2-, 3- oder 4-Pyridinyl, 2-, 3- oder 4-Chinolinyl, 1-, 3- oder 4-Isochinolinyl, 2-, 4- oder 5-Pyrimidyl oder 1,3,5-Trazinyl, ist und R¹⁰ ein Wasserstoffatom oder eine gerad- oder verzweigtkettige C₁ ― C₄-Alkylgruppe bedeutet, oder die Gruppe NR¹⁰-CO-R⁹ für ein cyclisches Amid oder Imid, vorzugsweise Phthalimid steht.

Gemäß einem ersten Aspekt umfasst die vorliegende Erfindung 3-Indolylmethylen-Verbindungen mit guten cytostatischen Eigenschaften mit folgender allgemeinen Formel: 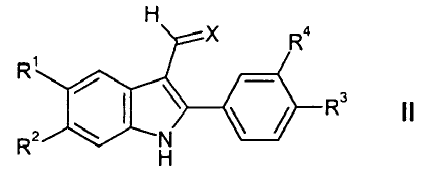 worin **R¹** und **R³** unabhängig voneinander
a) einen Wasserstoff, gerad- oder verzweigtkettige C₁- bis C₁₀-Alkyl-, -Alkenyl- oder -Alkinreste oder
b) gerad- oder verzweigtkettige C₁- bis C₆-Alkoxyreste oder
c) Halogenatome oder
d) ganz oder teilweise fluorierte Alkylreste mit 1 - 4 Kohlenstoffen darstellen,
**R²** Wasserstoff, einen gerad- oder verzweigtkettigen C₁- bis C₆-Alkylrest, ein Halogenatom, insbesondere Fluor oder Chlor, oder eine Alkoxygruppe mit 1 - 6 Kohlenstoffatomen bedeutet,
**R⁴** Wasserstoff, eine Hydroxygruppe oder eine Methoxygruppe bedeutet und
**X** die vorgenannten Bedeutungen hat.

Besonders geeignete Verbindungen sind die in der Tabelle 1 angegebenen 3-Indolylmethylen-Derivate.

Die Begriffe Alkyl, Alkenyl, Alkoxy, Alkinyl, Aryl etc. wie hierin verwendet weisen die Bedeutung auf, die diesen Begriffen in der wissenschaftlichen Literatur zugeordnet wird. Insofern in dieser Anmeldung auf eine spezielle Kettenlänge Bezug genommen wird, z.B. C₁-C₄ oder C₁-C₆, schliesst diese Definition alle in diesem Bereich liegenden Kettenlängen ein, z.B. C₁, C₂, C₃, C₄, C₅ oder C₆. Genauso sind alle in diese Definitionen fallenden Unterbereiche von der Erfindung mit umfasst, z.B. C₁-C₅, C₁-C₄, C₁-C₃, C₂-C₅ etc.

Die erfindungsgemäßen Verbindungen umfassen auch pharmakologisch verträgliche Additionssalze. Es handelt sich hierbei um die entsprechenden Additionssalze mit anorganischen und organischen Säuren. Als Additionssalze kommen insbesondere Hydrochloride, Hydrobromide, Acetate, Citrate, Oxalate, Tartrate und Methansulfonate in Betracht.

Die erfindungsgemäßen 3-Indolylmethylen-Derivate mit der allgemeinen Formel **II** sind insbesondere zur Herstellung von Arzneimitteln geeignet.

Die Erfindung betrifft außerdem pharmazeutische Präparate, die neben einem der vorgenannten 3-Indolylmethylen-Derivate mindestens einen pharmazeutisch verträglichen Träger enthalten.

Im Gegensatz zu den 2-Phenylindol-3-carbaldehyden zeigen die erfindungsgemäßen Derivate überraschenderweise keine oder nur geringe Hemmung der Tubulinpolymerisation. Dennoch hemmen sie schon in submikromolaren Konzentrationen das Wachstum von hormonunabhängigen Mammacarcinomzellen über eine Blockade des Zellzyklus in der G₂/M-Phase. Da diese Wirkstoffe keine Ähnlichkeit mit bisher bekannten Cyctostatika besitzen und auch nicht am Tubulin angreifen, muss hier ein neuartiger Wirkmechanismus vorliegen.

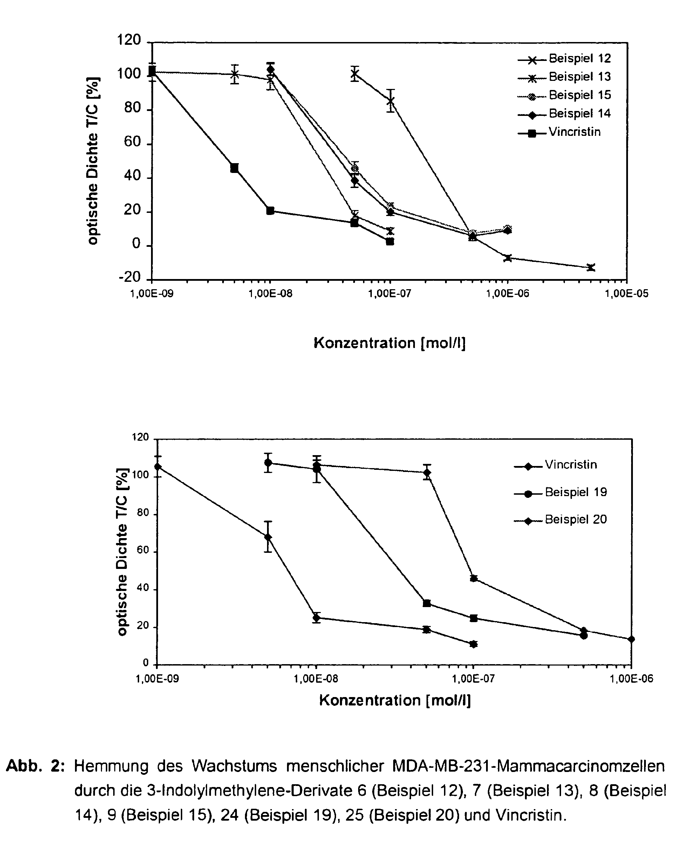

Die cytostatische Wirkung der erfindungsgemäßen Verbindungen wurde an zwei verschiedenen menschlichen Mammacarcinomzellinien in vitro bestimmt (M. Goldbrunner et al., J.Med.Chem. 1997,40,3525). Es handelt sich dabei um die hormonunabhängige MDA-MB-231-Zellinie und die estrogensensitive MCF-7-Linie. Die MDA-MB-231-Zellen wurden auch für die durchflusszytometrischen Analysen zur Zellzyklusverteilung herangezogen (M.J.J. Jaroszeski und R. Heller, Flow Cytometry Protocols, Humana Press, 1998, Totowa New Jersey). Besonders überraschend war, dass diese synthetischen Verbindungen häufig bereits im Konzentrationsbereich von 10 bis 100 nanomolar das Wachstum von menschlichen Tumorzellen stark hemmen, obwohl sie nur ein relativ niedriges Molekulargewicht aufweisen (MG ~ 320 - 500) (Tabelle 2 und 3, Abb. 2). Potente Therapeutika, die ebenfalls in den Zellzyklus eingreifen, besitzen Molekulargewichte im Bereich von 825 (Vincristin als Base) und 850 (Paclitaxel).

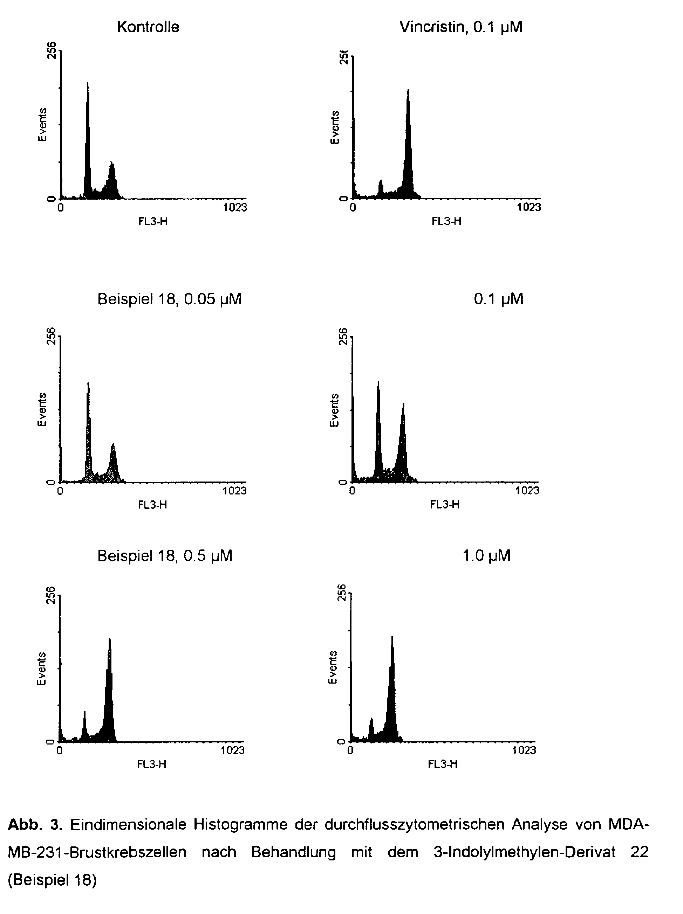

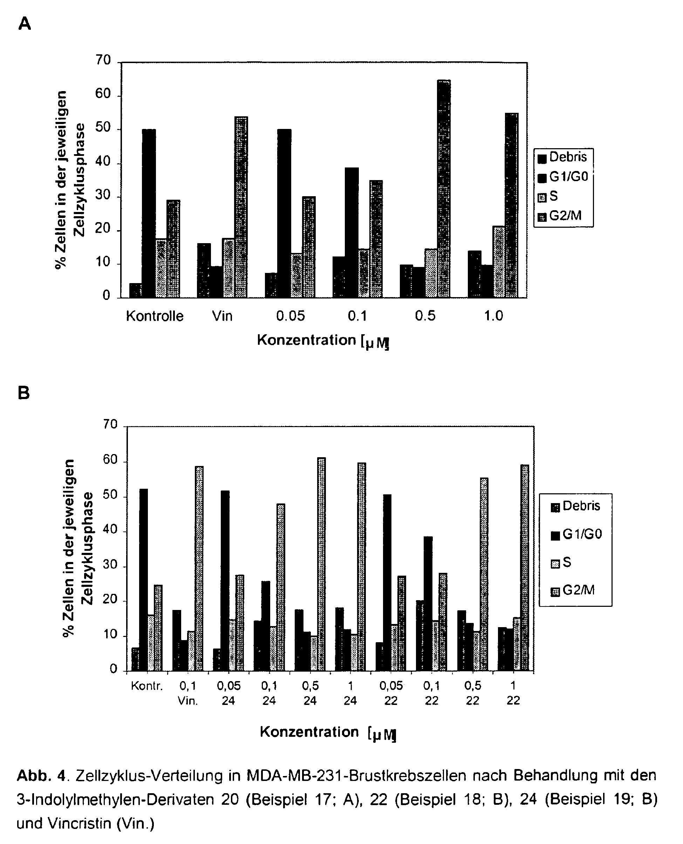

Die erfindungsgemäßen Verbindungen blockieren den Zellzyklus in der G₂/M-Phase in einer Konzentration von 0.1 bis 0.5 mikromolar in dem gleichen Ausmaß wie die Referenzverbindung Vincristin (Tabelle 4, Abb. 3 und 4). Eine Hemmung der Tubulinpolymerisation konnte nicht nachgewiesen werden, genauso wenig wie eine für Hemmstoffe der Cyclin-abhängigen Kinase 2 (CDK 2) charakteristische Blockade des Zellzyklus in der G₁-Phase. Daher muss den erfindungsgemäßen 3-Indolylmethylen-Derivate ein neuartiger Wirkmechanismus zugrundeliegen.

Gemäß bevorzugten Ausführungsformen ist **X** eine Methylengruppe mit zwei Substituenten, die unabhängig voneinander Carboxylgruppen, Carbonsäureamidgruppen, wobei die Aminogruppen entweder unsubstituiert oder mit C₁-C₆-Alkylgruppen substituiert sind, oder Nitrilgruppen sind,
ist **X** NR⁶, wobei R⁶ eine Hydroxygruppe oder ein C₁ - C₆-Alkylrest ist,
ist **X** eine Gruppierung N-NR⁷R⁸, wobei R⁷ ein Wasserstoffatom und R⁸ ein unsubstituierter oder substituierter, vorzugsweise ein- oder zweifach substituierter Phenylrest ist, oder
ist **X** eine Gruppierung N-NR¹⁰-CO-R⁹, wobei R⁹ eine Aminogruppe, ein unsubstituierter oder substituierter, vorzugsweise ein- oder zweifach substituierter Phenylrest oder Heteroarylrest, beispielsweise 2-, 3- oder 4-Pyridinyl, und R¹⁰ ein Wasserstoffatom bedeutet, oder die Gruppe NR¹⁰-CO-R⁹ für ein cyclisches aromatisches Amid oder Imid, beispielsweise Phthalimid steht.

Gemäß einer ebenfalls bevorzugten Ausführungsform stellt **R¹** einen Wasserstoff, einen gerad- oder verzweigtkettigen C₁- bis C₆-Alkylrest, eine Methoxygruppe oder ein Chlor- oder Fluoratom dar, **R²** ist Wasserstoff, eine Methoxygruppe oder ein Chlor- oder Fluoratom, **R³** bedeutet Wasserstoff, einen C₁- bis C₄-Alkylrest, eine Methoxygruppe oder eine Trifluormethylgruppe und **R⁴** Wasserstoff, eine Hydroxy- oder eine Methoxygruppe.

Es sei grundsätzlich angemerkt, dass der R₃ und R₄ tragende Phenylrest erfindungsgemäß auch durch einen heteroaromatischen Ring ersetzt werden kann, insbesondere durch einen Thiophen-Ring. Der Phenylenrest wird jedoch bevorzugt.

Die 3-Indolylmethylen-Derivate der Erfindung sind besonders bevorzugt aus der Gruppe ausgewählt ist, die aus
1) *[2-(4-Methoxyphenyl)-5-methyl-indol-3-yl]-methylenpropandinitril*
2) *[6-Chlor-2-(4-methoxyphenyl)-5-methyl-indol-3-yl]-methylenpropandinitril*
3) *[6-Methoxy-2-(4-methylphenyl)-indol-3-yl]-methylenpropandinitril*
4) *[5-Fluor-2-(4-methoxyphenyl)-indol-3-yl]methylenpropandinitril*
5) *[2-(4-Methoxyphenyl)-5-n-propyl-indol-3-yl]-methylenpropandinitril*
6) *[6-Isopropyl-2-(4-methoxyphenyl)-indol-3-yl]methylenpropandinitril*
7) *[5-n-Butyl-2-(4-methoxyphenyl)-indol-3-yl]-methylenpropandinitril*
8) *[2-(4-Methoxyphenyl)-5-n-pentyl-indol-3-yl]-methylenpropandinitril*
9) *[5-n-Hexyl-2-(4-methoxyphenyl)-indol-3-yl]-methylenpropandinitril*
10) *[6-Methoxy-2-(4-methoxyphenyl)-indol-3-yl]methylenpropandinitril*
11) *[2-(4-Methoxyphenyl)-indol-3-yl]-methylenpropandinitril*
12) *[5-Methoxy-2-(3-methoxyphenyl)-indol-3-yl]-methylenpropandinitril*
*13) {5-Butyl-2-[4-(trifluormethyl)-phenyl]-indol-3-yl}methylenpropandinitril*
14) *[5-Butyl-2-(4-methylphenyl)-indol-3-yl]-methylenpropandinitril*
15) *[5-Butyl-2-(4-ethylphenyl)-indol-3-yl]-methylenpropandinitril*
16) *{5-Pentyl-2-[4-(trifluormethyl)-phenyl]-indol-3-yl}-methylenepropandinitril*
*17) {5-Hexyl-2-[4-(trifluormethyl)phenyl]-indol-3yl}-methylenpropandinitril*
18) *3-[5-n-Butyl-2-(4-methoxyphenyl-)indol-3-yl]-2-cyanoacrylsäure*
*19) 5-n-Butyl-2-[4-(trifluormethyl)-phenyl]-indol-3-carbaldehyd-methylimin*
20) *5-n-Butyl-2-(4-methoxyphenyl)-indol-3-carbaldehyd-oxim*
*21) 6-Methoxy-2-(4-methoxyphenyl)-indol-3-carbaldehyd-phenylhydrazon*
*22) 5-n-Butyl-2-(4-methoxyphenyl)-indol-3-carbaldehyd-benzoylhydrazon*
23) *5-n-Butyl-2-(4-methoxyphenyl)-indol-3-carbaldehyd-(4-chlorbenzoyl)hydrazon*
*24) 5-n-Butyl-2-(4-methoxyphenyl)-indol-3-carbaldehyd-nicotinylhydrazon*
25) *5-Butyl-2-[4-(trifluormethyl)-phenyl]-indol-3-carbaldehyd-nicotinylhydrazone*
*26) 5-Butyl-2-(4-ethylphenyl)-indol-3-carbaldehyd-nicotinylhydrazon*
*27) 5-n-Butyl-2-[4-(trifluormethyl)-phenyl]-indol-3-carbaldehyd-picolinylhydrazon*
*28) 5-Butyl-2-[4-(trifluormethyl)-phenyl]-indol-3-carbaldehyd-isonicotinylhydrazon*
*29) N-{[5-n-Butyl-2(4-methoxyphenyl)-indol-3-yl]methylenamino}-phthalimid,* und
*30) 5-Butyl-2-[4-(trifluormethyl)-phenyl]-indol-3-carbaldehyd-semicarbazon*
besteht.

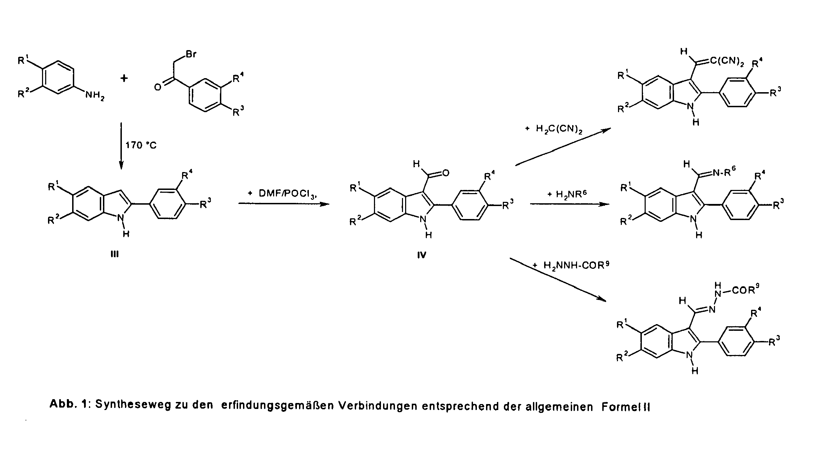

Die Synthese der erfindungsgemäßen 3-Indolylmethylen-Derivate soll im Folgenden exemplarisch dargestellt werden: (vgl. Abb. 1)

Bei der Synthese der erfindungsgemäßen 3-Indolylmethylen-Derivate mit der allgemeinen Formel 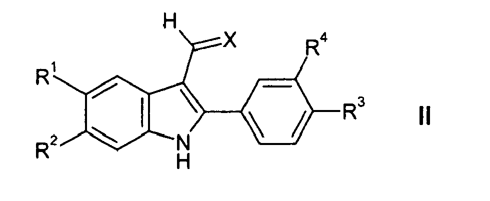 wird von einer Verbindung der allgemeinen Formel 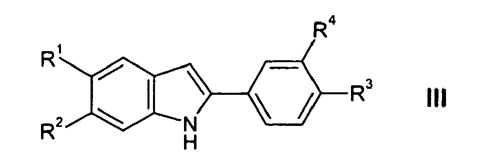 ausgegangen, die durch übliche Indolsynthesen, z. B. durch das unten angegebene Bischler-Verfahren, zugänglich ist. Durch Einführung einer Formylgruppe in Position 3 des Indols erhält man als wichtiges Zwischenprodukt die Verbindung **IV** mit der allgemeinen Formel

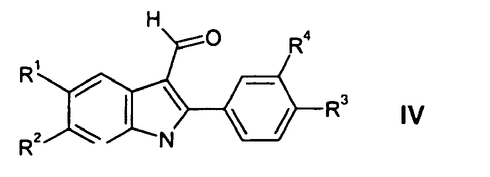

Die Formylgruppe wird in bekannter Weise durch Umsetzung des 2-Phenylindols mit aktivierten Derivaten der Ameisensäure, beispielsweise Dialkylformamid, vorzugsweise Dimethylformamid, eingeführt. Zur Aktivierung eignen sich Phosphoroxychlorid und Phosphorpentachlorid.

Die substituierten 2-Phenylindol-3-carbaldehyde der allgemeinen Formel **IV** werden mit C-H-aciden Verbindungen, primären Aminen, Hydroxylamin, Alkoxyaminen oder Hydrazin-Derivaten zu den erfindungsgemäßen 3-Indolylmethylen-Derivaten der allgemeinen Formel 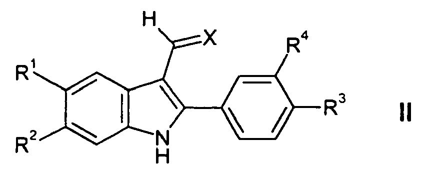 umgesetzt. Die entsprechenden Kondensationreaktionen werden in Gegenwart saurer oder basischer Katalysatoren durchgeführt. Geeignet sind sekundäre und tertiäre Amine, beispielsweise Piperidin, verdünnte Alkalilösungen, organische und anorganische Säuren, sowie entsprechende Ionenaustauscher.

In einem weiteren Aspekt ist die vorliegende Erfindung auf die Verwendung einer Verbindung, die den Strukturteil 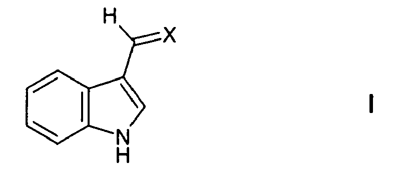 umfasst, zur Herstellung eines Arzneimittels für die cytostatische Therapie gerichtet, wobei **X** die oben angegebene Bedeutung aufweist.

Die Verwendung der 3-Indolylmethylen-Derivate erfolgt vorzugsweise für die cytostatische Therapie, besonders bevorzugt zur Behandlung von Krebs- und Viruserkrankungen, von hyperproliferativen Erkrankungen sowie Autoimmunkrankheiten.

Hier sei insbesondere auf hormonunabhängiges Mammacarcinom, hormonrefraktäres Prostatacarcinom, bösartige Tumore von Niere und Colon, nicht-kleinzelliges Lungencarcinom und chronisch lymphatische Leukämie hingewiesen.

Wie bereits oben angesprochen umfasst die vorliegende Erfindung auch eine pharmazeutische Zusammensetzung, die die erfindungsgemäßen Wirkstoffe umfasst.

Die Wirkstoffe der vorliegenden Erfindung werden dazu vorzugsweise in Form einer pharmazeutischen Zusammensetzung verwendet, in der sie mit geeigneten Trägem oder Trägerstoffen in solchen Dosen vermischt werden, dass die Erkrankung behandelt oder zumindest gelindert wird. Eine derartige Zusammensetzung kann Verdünnungsmittel, Füllmaterialien, Salze, Puffer, Stabilisatoren, Solubilisierungsmittel und andere Materialien enthalten, die dem Fachmann bekannt sind. Der Begriff "pharmazeutisch verträglich" ist als ein nichttoxisches Material definiert, das die Wirksamkeit des aktiven Wirkstoffes nicht stört. Die Auswahl des Trägers hängt vom Verabreichungsweg ab.

Diese Verabreichung kann grundsätzlich über jeden Weg erfolgen, beispielsweise als perorale, topische (z.B. bei Hautmetastasen) und parenterale Verabreichung, einschließlich intramuskulärer, subkutaner, intravenöser, intraperitonealer oder intranasaler Injektionen.

Die pharmazeutische Zusammensetzung kann zusätzlich weitere Mittel enthalten, die die Aktivität des Wirkstoffes steigern oder dessen Aktivität oder Verwendung bei der Behandlung ergänzen. Derartige zusätzliche Mittel können in der pharmazeutischen Zusammensetzung enthalten sein, um eine synergistische oder zusätzliche Wirkung zu erzielen oder um Nebenwirkungen bzw. unerwünschte Wirkungen zu minimieren. Vorzugsweise sind diese Mittel aus Paclitaxel, Docetaxel, Irinotecan und Gemcitabine ausgewählt.

Der Begriff der pharmazeutischen Zusammensetzung umfasst auch Kombinationen des erfindungsgemäßen Wirkstoffes und weiterer Mittel. Eine solche Kombination bedeutet, dass die Wirkstoffe zwar zur Behandlung derselben Krankheit bei demselben Patienten eingesetzt werden, dass die Verabreichung jedoch nach unterschiedlichen Applikationsschemen erfolgt. Beispielsweise können die zusätzlichen Wirkstoffe vor, gleichzeitig mit oder nach der Verabreichung des erfindungsgemäßen Wirkstoffes verabreicht werden. Beispielsweise können die verschiedenen Wirkstoffe in einem "Kit-of-parts" kombiniert vorliegen.

Techniken zur Formulierung bzw. Zubereitung und Verabreichung der Verbindungen der vorliegenden Anmeldung sind in "Remington's Pharmaceutical Sciences", Mack Publishing Co., Easton, PA, neueste Ausgabe (oder vergleichbaren Fachbüchern) zu finden.

Die nachfolgend dargestellten Beispiele und Abbildungen dienen zur näheren Erläuterung der Erfindung.

In den Abbildungen zeigt
Abb. 1 einen Syntheseweg der erfindungsgemäßen Verbindungen;
Abb. 2 die Hemmung des Wachstums menschlicher MDA-MB-231-Mammacarcinomzellen durch die 3-Indolylmethylene-Derivate 6 (Beispiel 12), 7 (Beispiel 13), 8 (Beispiel 14), 9 (Beispiel 15), 24 (Beispiel 19), 25 (Beispiel 20) und Vincristin;
Abb. 3 eindimensionale Histogramme der durchflusszytometrischen Analyse von MDA-MB-231-Brustkrebszellen nach Behandlung mit dem 3-Indolylmethylen-Derivat 22 (Beispiel 18).
Abb. 4 die Zellzyklus-Verteilung in MDA-MB-231-Brustkrebszellen nach Behandlung mit den 3-Indolylmethylen-Derivaten 20 (Beispiel 17; A), 22 (Beispiel 18; B), 24 (Beispiel 19; B) und Vincristin

### Synthesevorschriften und analytische Daten

### Allgemeine Vorschrift zur Herstellung der substituierten 2-Phenylindole entsprechend Formel III

Eine Lösung von 50 mmol des entsprechenden Anilins in 12 ml N,N-Dimethylanilin wird auf 170 °C erhitzt. Bei dieser Temperatur tropft man innerhalb einer Stunde 20 mmol des bromierten Phenonderivats, welches in 250 ml Xylol gelöst ist, zu. Anschließend wird noch drei Stunden bei 170°C am Sieden gehalten. Nach dem Abkühlen wird die breiige Suspension mit 500 ml 2N Salzsäure und 500 ml Essigsäureethylester versetzt, wobei sich eine braune Emulsion bildet. Die wässrige Phase wird abgetrennt und dreimal mit 250 ml Essigsäureethylester extrahiert. Die vereinigten organischen Phasen werden mit 300 ml Wasser gewaschen, über Magnesiumsulfat getrocknet und im Vakuum eingeengt.

Die Reinigung erfolgt durch Säulenchromatographie an Kieselgel 60 mit unterschiedlichen Laufmittelgemischen auf Methylenchloridbasis und anschliessender Umkristallisation aus Ethanol.

### Allgemeine Vorschrift zur Herstellung der substituierten 2-Phenylindol-3-carbaldehyde entsprechend Formel IV

Unter Stickstoff und Feuchtigkeitsausschluss werden 20 mmol Phosphoroxychlorid bei etwa 15 °C zu 15 mmol trockenem Dimethylformamid getropft, man lässt 10 Minuten nachrühren, anschließend wird eine Lösung aus 2 mmol des entsprechenden Indols in ca. 10 mL trockenem Dimethylformamid zugegeben, wobei die Temperatur durch Kühlung im Bereich von 10-20 °C gehalten wird. Anschließend wird auf 70 °C erhitzt und 2 Stunden lang bei dieser Temperatur gerührt. Diese Reaktionsmischung wird auf 100 mL Eis gegossen und mittels 40%iger NaOH neutralisiert, wobei hierbei eine Temperatur von 20 °C nicht überschritten werden soll, da sich sonst ein grün-blauer Farbstoff bildet, der sich später vom Produkt nicht mehr abtrennen lässt.

Die wässrige Phase wird abgetrennt und viermal mit 100 mL Chloroform extrahiert. Die vereinigten organischen Phasen werden mit Wasser und gesättigter NaCl-Lösung gewaschen, über Magnesiumsulfat getrocknet und im Vakuum eingeengt.

Die Reinigung erfolgt teils durch Säulenchromatographie an Kieselgel 60, teils durch Umkristallisation aus trockenem Methanol.

### Beispiel 1:

### [2-(4-Methoxyphenyl)-5-methyl-indol-3-yl]-carbaldehyd

Reinigung durch Umkristallisation aus trockenem Methanol (+4 °C).
Grauer Feststoff; Ausbeute: 79 %
Schmp.: 242 °C
C₁₇H₁₅NO₂ (265.31)

| | |
|---|---|
| IR (KBr): | ν (cm⁻¹) = 3149 (m; br; N-H); 1619 (s; C=O) |
| ¹H-NMR (DMSO-d₆): | δ (ppm) = 2.42 (s; 3H; -CH₃); 3.86 (s; 3H; -OCH₃); 7.09 (dd; ³J = 9 Hz; ⁴J = 1 Hz; 1H; Indol-H⁶); 7.15 und 7.71 (AA'BB'; ³J = 9 Hz; 4H; Phenyl-H); 7.36 (d; ³J = 8 Hz; 1 H; Indol-H⁷ 8.01 (s; 1H; Indol-H⁴); 9.92 (s; 1 H; Formyl-H); 12.17 (s; br; 1H; N-H) |

### Beispiel 2:

### [6-Chlor-2-(4-methoxyphenyl)-5-methyl-indol-3-yl]-carbaldehyd

Reinigung durch Umkristallisation aus trockenem Methanol (+4 °C).
Grauer Feststoff; Ausbeute: 28 %
Schmp.:287°C
C₁₇H₁₄NO₂Cl (299.76)

| | |
|---|---|
| IR (KBr): | ν (cm⁻¹) = 3127 (m; br; N-H); 1610 (s; C=O) |
| ¹H-NMR (DMSO-d₆): | δ (ppm) = 2.43 (s; 3H; -CH₃); 3.33 (s; 3H; -OCH₃); 7.16 und 7.73 (AA'BB'; ³J = 9 Hz; 4H; Phenyl-H); 7.49 (s; 1H; Indol-H⁴); 8.14 (s; 1H; Indol-H⁷); 9.92 (s; 1H; Formyl-H); 12.31 (s; br; 1H; N-H) |

### Beispiel 3:

### [6-Methoxy-2-(4-methylphenyl)-indol-3-yl]-carbaldehyd

Reinigung durch Umkristallisation aus trockenem Methanol (+4 °C).
Hellblauer Feststoff; Ausbeute: 60 %
Schmp.: 250 °C
C₁₇H₁₅NO₂ (265.31)

| | |
|---|---|
| IR (KBr): | v (cm⁻¹) = 3114 (m; br; N-H); 1624 (s; C=O) |
| ¹H-NMR (DMSO-d₆): | δ (ppm) = 2.42 (s; 3H; -CH₃); 3.81 (s; 3H; -OCH₃); 6.87 (dd; ³J = 8 Hz; ⁴J = 2 Hz; 1H; Indol-H⁵); 6.95 (d; ⁴J = 2 Hz; 1 H; Indol-H⁷); 7.40 und 7.63 (AA'BB'; ³J = 9 Hz; 4H; Phenyl-H); 8.05 (d; ³J = 8 Hz; 1H; Indol-H⁴); 9.91 (s; 1H; Formyl-H); 12.17 (s; 1H; N-H) |

### Beispiel 4:

### [5-Isopropyl-2-(4-methoxyphenyl)-indol-3-yl]-carbaldehyd

Reinigung durch Umkristallisation aus trockenem Methanol (+4 °C).
Hellblauer Feststoff; Ausbeute: 54 %
Schmp.: 217 °C
C₁₉H₁₉NO₂ (293.37)

| | |
|---|---|
| IR (KBr): | ν (cm⁻¹) = 3436 (m; br; N-H); 1617 (s; C=O) |
| ¹H-NMR (DMSO-d₆): | δ (ppm) = 1.25 (d; ³J = 7Hz; 6H; -CH-(CH₃)₂); 2.99 (septett; ³J = 7Hz; 1H; -CH-(CH₃)₂); 3.85 (s; 3H; -OCH₃); 7.15 und 7.69 (AA'BB'; ³J = 9 Hz; 4H; Phenyl-H); 7.16 (d; ³J = 8 Hz; 1H; Indol-H⁶); 7.38 (d; ³J = 8 Hz; 1H; Indol-H⁷); 8.05 (s; 1H; Indol-H⁴); 9.92 (s; 1H; Formyl-H); 12.16 (s; 1H; N-H) |

### Beispiel 5:

### [5-n-Butyl-2-(4-methoxphenyl)-indol-3-yl]-carbaldehyd

Reinigung durch Umkristallisation aus trockenem Methanol (+4 °C).
Orange Kristalle; Ausbeute: 44 %
Schmp.: 210°C
C₂₀H₂₁NO₂ (307.39)

| | |
|---|---|
| IR (KBr): | v (cm⁻¹) = 3144 (m; br; N-H); 1623 (s; C=O) |
| ¹H-NMR (DMSO-d₆): | δ (ppm) = 0.92 (t; ³J = 7 Hz; 3H; -(CH₂)₃-CH₃); 1.34 (sextett; ³J = 7 Hz; 2H; -(CH₂)₂-CH₂-CH₃); 1.63 (quin; ³J = 7 Hz; 2H; -CH₂-CH₂-CH₂-CH₃); 2.73 (t; ³J = 7 Hz; 2H; -CH₂-(CH₂)₂-CH₃); 3.89 (s; 3H; -OCH₃); 7.22-7.24 (m; 1H; Indol-H⁶); 7.21 und 7.67 (AA'BB'; ³J = 9 Hz; 4H; Phenyl-H); 7.22-7.24 (m; 1H; Indol-H⁶); 7.52 (d; J³ = 8 Hz; 1H; Indol-H⁷); 7.57 (s; 1H; Indol-H⁴); 8.71 (s; 1H; Formyl-H); 13.19 (s; 1H; -NH) |

### Beispiel 6:

### 5-n-Butyl-2-(4-trifluormethylphenyl)-indol-3-carbaldehyd

Reinigung durch Säulenchromatographie mit Methylenchlorid und Umkristallisation aus trockenem Methanol (+4 °C).
Gelber Feststoff; Ausbeute: 79 %
Schmp.: 230-231 °C (Cu-Block)
C₂₀H₁₈NO (345,36)

| | |
|---|---|
| IR (KBr): | ν (cm⁻¹) = 3144 (m; br; N-H); 1615 (s; C=O) |
| ¹H-NMR (DMSO-d₆): | δ (ppm) = 0,91 (t; ³J = 7,3Hz; 3H; -CH₂-CH₂-**CH**₂-CH₃); 1,33 (m; 2H; -CH₂-CH₂-**CH₂**-CH₃); 1,59 (m; 2H; -CH₂**-CH₂**-CH₂-CH₃); 2,71 (t; ³J = 7,5Hz; 2H; -**CH**₂-CH₂-CH₂-CH₃); 7,17 (dd; ³J = 8,4Hz; ⁴J = 1,5Hz; 1H; Indol-H⁶); 7,45 (d; ³J = 8,3Hz; 1H; Indol-H⁷); 7,96 und |
| | 8,01 (AA'BB'; ³J = 8,6Hz; 4H; Phenyl-H); 8,05 (s; 1H; Indol-H⁴); 9,98 (s;1H; Formyl-H); 12,50 (s; 1H; Indol-NH) |

### Beispiel 7:

### [2-(4-Methoxyphenyl)-5-n-pentyl-indol-3-yl]-carbaldehyd

Reinigung durch Umkristallisation aus trockenem Methanol (+4 °C).
Orange Kristalle; Ausbeute: 60 %
Schmp.: 182°C
C₂₁H₂₃NO₂ (321.42)

| | |
|---|---|
| IR (KBr): | ν (cm⁻¹) = 3144 (m; br; N-H); 1617 (s; C=O) |
| ¹H-NMR (DMSO-d₆): | δ (ppm) = 0.87 (t; ³J = 7 Hz; 3H; -(CH₂)₄-CH₃); 1.30 (m; 4H; -(CH₂)₂-(CH₂)₂-CH₃); 1.62 (m; 2H; -CH₂-CH₂-(CH₂)₂-CH₃); 2.68 (t; ³J = 7Hz; 2H; -CH₂-(CH₂)₃-CH₃); 3.86 (s; 3H; -OCH₃); 7.10 (dd; ⁴J = 2 Hz; ³J = 8 Hz; 1H; Indol-H⁶); 7.16 und 7.70 (AA'BB'; ³J = 9 Hz; 4H; Phenyl-H); 7.38 (d; J³ = 8 Hz; 1 H; Indol-H⁷); 8.00 (s; 1H; Indol-H⁴); 9.92 (s; 1H; Formyl-H); 12.18 (s; 1H; -NH) |

### Beispiel 8:

### [5-n-Hexyl-2-(4-methoxyphenyl)-indol-3-yl]-carbaldehyd

Reinigung durch Umkristallisation aus trockenem Methanol (+4 °C).
Orange Kristalle; Ausbeute: 60 %
Schmp.: 176°C
C₂₂H₂₅NO₂ (335.45)

| | |
|---|---|
| IR (KBr): | ν (cm⁻¹) = 3156 (m; br; N-H); 1626 (s; C=O) |
| ¹H-NMR (DMSO-d₆): | δ (ppm) = 0.88 (t; ³J = 7 Hz; 3H; -(CH₂)₅-CH₃); 1.29 (m; 6H; -(CH₂)₂-(CH₂)₃-CH₃); 1.61 (m; 2H; -CH₂-CH₂-(CH₂)₃-CH₃); 2.68 (t; ³J = 7 Hz; 2H; -CH₂-(CH₂)₄-CH₃); 3.86 (s; 3H; -OCH₃); 7.10 (d; ³J= 8 Hz; 1H; Indol-H⁶); 7.15 und 7.70 (AA'BB'; ³J = 9 Hz; 4H; Phenyl-H); 7.37 (d; ³J= 8 Hz; 1H; Indol-H⁷); 8.00 (s; 1H; Indol-H⁴); 9.92 (s; 1H; Formyl-H); 12.17 (s; 1 H; -NH) |

### Allgemeine Vorschrift zur Herstellung der substituierten (2-Phenylindol-3-yl)-methylenpropandinitrile

Eine Suspension aus 50 mmol des entsprechenden Formylindols und 55 mmol Malonsäuredinitril in 5 mL Ethanol, versetzt mit 2-3 Tropfen Piperidin, wird zwei Stunden zum Sieden erhitzt. Nach dem Abkühlen gibt man langsam 50 mL Wasser zu und saugt den kristallinen Niederschlag ab. Das getrocknete Produkt wird durch Umkristallisation aus Ethanol gereinigt.

### Beispiel 9:

### [2-(4-Methoxyphenyl)-5-methyl-indol-3-yl]-methylenpropandinitril

Darstellung aus [2-(4-Methoxyphenyl)-5-methyl-indol-3-yl]-carbaldehyd und Malonsäuredinitril, Reinigung durch Umkristallisation aus Ethanol (+4 °C).
Gelber Feststoff; Ausbeute: 71 %
Schmp.: 260 °C
C₂₀H₁₅N₃O (313.36)

| | |
|---|---|
| IR (KBr): | v (cm⁻¹) = 3254 (m; br; -NH); 2218 (s;-CN) |
| ¹H-NMR (DMSO-d₆): | δ (ppm) = 2.43 (s; 3H; -CH₃); 3.84 (s; 3H; -OCH₃); 6.94-7.01 (m 2H; Indol-H^{7,6}); 7.45 und 7.53 (AA'BB'; ³J = 9 Hz; 4H; Phenyl-H); 7.86 (s; 1H; Vinyl-H); 8.00 (s; 1H; Indol-H⁴); 12.86 (s; br; 1H; N-H) |

### Beispiel 10:

### [6-Chlor-2-(4-methoxyphenyl)-5-methyl-indol-3-yl]-methylenpropandinitril

Darstellung aus [6-Chlor-2-(4-methoxyphenyl)-5-methyl-indol-3-yl]-carbaldehyd und Malonsäuredinitril, Reinigung durch Umkristallisation aus Ethanol (+4 °C).
Gelber Feststoff; Ausbeute: 47 %
Schmp.: 234 °C
C₂₀H₁₄N₃OCl (347.80)

| | |
|---|---|
| IR (KBr): | v (cm⁻¹) = 3388 (m; br; -NH); 2216 (s;-CN) |
| ¹H-NMR (DMSO-d₆): | δ (ppm) = 2.45 (s; 3H; -CH₃)₃ 3.87 (s; 3H; -OCH₃); 7.20 und 7.60 (AA'BB'; ³J = 9 Hz; 4H; Phenyl-H); 7.54 (s; 1H; Indol-H⁴); 7.96 (s; 1H; Indol-H⁷); 8.00 (s; 1H; Vinyl-H); 12.31 (s; br; 1H; N-H) |

### Beispiel 11:

### [6-Methoxy-2-(4-methylphenyl)-indol-3-yl]-methylenpropandinitril

Darstellung aus 6-Methoxy-2-(4-methylphenyl)-indol-3-yl]-carbaldehyd und Malonsäuredinitril, Reinigung durch Umkristallisation aus Ethanol (+4 °C).
Gelber Feststoff; Ausbeute: 47 %
Schmp.: 258 °C
C₂₀H₁₅N₃O (313.36)

| | |
|---|---|
| IR (KBr): | ν (cm⁻¹) = 3299 (m; br; -NH); 2221 (s;-CN) |
| ¹H-NMR (DMSO-d₆): | δ (ppm) = 2.45 (s; 3H; -CH₃); 3.87 (s; 3H; -OCH₃); 7.14 (dd; ³J = 8 Hz; ⁴J = 2 Hz; 1H; Indol-H⁵); 7.43 (d; ⁴J = 2 Hz; 1H; Indol-H⁷); 7.20 und 7.60 (AA'BB'; ³J = 9 Hz; 4H; Phenyl-H); 7.90 (s, 2H, Vinyl-H und Indol-H⁴); 12.86 (s; 1H; N-H) |

### Beispiel 12:

### [6-Isopropyl-2-(4-methoxyphenyl)-indol-3-yl]methylenpropandinitril

Darstellung aus [6-Isopropyl-2-(4-methoxyphenyl)-indol-3-yl]-carbaldehyd und Malonsäuredinitril, Reinigung durch Umkristallisation aus Ethanol (+4 °C).
Gelber Feststoff; Ausbeute: 33 %
Schmp.: 145°C
C₂₂H₁₉N₃O (341.41)

| | |
|---|---|
| IR (KBr): | ν (cm⁻¹) = 3278 (m; br; -NH); 2216 (s;-CN) |
| ¹H-NMR (DMSO-d₆): | δ (ppm) = 1.27 (d; ³J = 7 Hz; 6H; -CH-(CH₃)₂); 3.01 (septett; ³J = 7 Hz; 1H; -CH-(CH₃)₂); 3.86 (s; 3H; -OCH₃); 7.20 und 7.58 (AA'BB'; ³J = 9 Hz; 4H; Phenyl-H); 7.18- 7.26 (m; 1H; Indol-H⁶); 7.43 (d; ³J = 8 Hz; 1H; Indol-H⁷); 7.87 (s; 1H; Indol-H⁴); 7.99 (s; 1H; Vinyl-H); 12.86 (s; br; 1H; N-H) |

### Beispiel 13:

### [5-n-Butyl-2-(4-methoxyphenyl)-indol-3-yl]-methylenpropandinitril

Darstellung aus [5-*n*-Butyl-2-(4-methoxyphenyl)-indol-3-yl]-carbaldehyd und Malonsäuredinitril, Reinigung durch Umkristallisation aus Ethanol (+4 °C).
Gelber Feststoff; Ausbeute: 78 %
Schmp.: 154 °C
C₂₃H₂₁N₃O (355.44)

| | |
|---|---|
| IR (KBr): | v (cm⁻¹) = 3278 (m; br; -NH); 2216 (s;-CN) |
| ¹H-NMR (DMSO-d₆): | δ (ppm) = 0.90 (t; ³J = 7 Hz; 3H; -(CH₂)₃-CH₃); 1.33 (sextett; ³J = 7Hz; 2H; -(CH₂)₂-CH₂-CH₃); 1.64 (quin; ³J = 7Hz; 2H; -CH₂-CH₂-CH₂-CH₃); 2.71 (t; ³J = 7Hz; 2H; -CH₂-(CH₂)₂-CH₃); 3.87 (s; 3H; -OCH₃); 7.20 und 7.59 (AA'BB'; ³J = 9 Hz; 4H; Phenyl-H); 7.19 (d; ³J = 8 Hz; 1H; Indol-H⁶); 7.52 (d; J³ = 8 Hz; 1H; Indol-H⁷); 7.88 (s; 1H; Indol-H⁴); 7.93 (s; 1H; Vinyl-H); 12.82 (s; 1H; -NH) |

### Beispiel 14:

### [2-(4-Methoxyphenyl)-5-n-pentyl-indol-3-yl]-niethylenpropandinitril

Darstellung aus [2-(4-Methoxyphenyl)-5-*n*-pentyl-indol-3-yl]-carbaldehyd und Malonsäuredinitril, Reinigung durch Umkristallisation aus Ethanol (+4°C).
Gelber Feststoff; Ausbeute: 75 %
Schmp.: 158°C
C₂₄H₂₃N₃O (369.47)

| | |
|---|---|
| IR (KBr): | ν (cm⁻¹) = 3342 (m; br; -NH); 2214 (s;-CN) |
| ¹H-NMR (DMSO-d₆): | δ (ppm) = 0.86 (t; ³J = 7 Hz; 3H; -(CH₂)₄-CH₃); 1.26 (m; 4H; -(CH₂)₂-(CH₂)₂-CH₃); 1.64 (m; 2H; -CH₂-CH₂-(CH₂)₂-CH₃); 2.70 (t; ³J = 7Hz; 2H; -CH₂-(CH₂)₃-CH₃); 3.87 (s; 3H; -OCH₃); 7.18 (d; ³J = 8 Hz; 1H; Indol-H⁶); 7.20 und 7.60 (AA'BB'; ³J = 9 Hz; 4H; Phenyl-H); 7.43 (d; ³J = 8 Hz; 1H; Indol-H⁷); 7.88 (s; 1H; Indol-H⁴); 7.93 (s; 1H; Vinyl-H); 12.87 (s; br; 1H; -NH) |

### Beispiel 15:

### [5-n-Hexyl -2-(4-methoxyphenyl)-indol-3-yl]-methylenpropandinitril

Darstellung aus [5-n-Hexyl-2-(4-methoxyphenyl)-indol-3-yl]-carbaldehyd und Malonsäuredinitril, Reinigung durch Umkristallisation aus Ethanol (+4 °C).
Gelber Feststoff; Ausbeute: 78 %
Schmp.: 131°C
C₂₅H₂₅N₃O (383.49)

| | |
|---|---|
| IR (KBr): | ν (cm⁻¹) = 3259 (m; br; -NH); 2217 (s;-CN) |
| ¹H-NMR(DMSO-d₆): | δ (ppm) = 0.85 (t; ³J = 7 Hz; 3H; -(CH₂)₃-CH₃); 1.28 (m; 2H; -(CH₂)₂-CH₂-CH₃); 1.64 (m; 2H; -CH₂-CH₂-CH₂-CH₃); 2.70 (t; ³J = 7Hz; 2H; -CH₂-(CH₂)₂-CH₃); 3.87 (s; 3H; -OCH₃); 7.16 (d; ⁴J = 1Hz; 1H; Indol-H⁶); 7.20 und 7.59 (AA'BB'; ³J = 9 Hz; 4H; Phenyl-H); 7.44 (d; ³J = 8 Hz; 1H; Indol-H⁷); 7.88 (s; 1H; Indol-H⁴); 7.93 (s; 1H; Formyl-H); 12.85 (s; br; 1H; -NH) |

### Allgemeine Vorschrift zur Herstellung der substituierten 2-Phenylindol-3-carbaldehyd-N-methylimine

Der entsprechend substituierte Indol-3-carbaldehyd (1 mmol) wird in 35 ml Methylenchlorid unter Stickstoff vorgelegt und mit 7 mmol Methylamin, als 33%ige ethanolische Lösung, versetzt. Man lässt über Nacht bei 40°C rühren. Danach gibt man 35 ml Wasser zu und schüttelt mit mehreren 20 ml Portionen Chloroform aus. Die vereinigten organischen Phasen werden mit Wasser und gesättigter NaCl-Lösung gewaschen, über Natriumsulfat getrocknet und das Lösungsmittel im Vakuum entfernt. Das getrocknete Produkt wird durch Umkristallisation aus Ethanol gereinigt.

### Beispiel 16

### 5-n-Butyl-2-[4-(trifluormethyl)phenyl]-indol-3-carbaldehyd-methylimin

Darstellung aus 5-n-Butyl-2-[4-(trifluormethyl)phenyl]-indol-3-carbaldehyd und Methylamin.
Weißer Feststoff; Ausbeute: 63 %
Schmp.:186°C
C₂₁H₂₁F₃N₂ (358,41)

| | |
|---|---|
| IR (KBr): | ν (cm⁻¹) =1630 (m; -C=N-) |
| ¹H-NMR (DMSO-d₆): | δ (ppm) = 0,91 (t; ³J = 7,3Hz; 3H; -CH₂-CH₂-CH₂-**CH₃**); 1,33 (sextett; ³J = 7,4Hz; 2H; -CH₂-CH₂-**CH₂**-CH₃);1,59 (quintet; ³J = 7,5Hz; 2H; -CH₂-CH₂-**CH**₂-CH₃); 2,67 (t; ³J = 7,6Hz; 2H; **-CH₂**-CH₂-CH₂-CH₃); 3,43 (d; ⁴J = 1,2Hz; 3H; =N-CH₃); 7,08 (dd; ³J = 8,3Hz; ⁴J = 1,6Hz; 1H; Indol-H⁶); 7,36 (d; ³J = 8,3Hz; 1H; Indol-H⁷); 7,85 und 7,93 (AA'BB'; ³J = 8,3Hz; 4H; Phenyl-H); 8,14 (s; 1H; Indol-H⁴); 8,49 (s; 1H; Azomethin-H); 11,85 (s; 1H; Indol-NH) |

### Allgemeine Vorschrift zur Herstellung der substituierten 2-Phenylindol-3-carbaldehyd-oxime

In einem 50 ml Kolben werden 1,2 mmol Hydroxylammoniumchlorid in 8 ml Wasser gelöst. Dann setzt man 1 mmol des entsprechenden Formylindols, in 8 ml Ethanol und 1,6 mmol Natriumacetat zu. Die Reaktionsmischung wird unter Rückfluss 3 Stunden gerührt und bei Raumtemperatur noch über Nacht. Danach extrahiert man mit mehreren Portionen Methylenchlorid. Die vereinigten organischen Phasen werden mit Wasser gewaschen, über Natriumsulfat getrocknet und das Lösungsmittel im Vakuum entfernt.

### Beispiel 17

### 5-n-Butyl-2-(4-methoxyphenyl)-indol-3-carbaldehyd-oxim

Darstellung aus 5-n-Butyl-2-(4-methoxyphenyl)-indol-3-carbaldehyd und Hydroxylammoniumchlorid. Reinigung durch Umkristallisation aus 99%igem Ethanol (+4 °C).
Weißer Feststoff; Ausbeute: 67 %
Schmp.:138 °C
C₂₀H₂₂N₂O₂ (322,41)

| | |
|---|---|
| IR (KBr): | v (cm⁻¹) = 3315 (s; br; N-OH) |
| ¹H-NMR (DMSO-d₆): | δ (ppm) = 0,90 (t; ³J = 7,3Hz; 3H; -CH₂-CH₂-CH₂-**CH**₃); 1,32 (sextett; ³J = 7,3Hz; 2H; -CH₂-CH₂**-CH₂**-CH₃); 1,58 (quintet; ³J = 7,5Hz; 2H; -CH₂-**CH**₂-CH₂-CH₃); 2,65 (t; ³J = 7,5Hz; 2H; -**CH**₂-CH₂-CH₂-CH₃); 3,84 (s; 3H; -OCH₃); 7,02 (dd; ³J = 8,3Hz; ⁴J = 1,5Hz; 1H; Indol-H⁶); 7,13 und 7,51 (AA'BB'; ³J = 8,7Hz; 4H; Phenyl-H); 7,30 (d; ³J = 8,2Hz; 1 H; Indol-H⁷); 7,88 (s; 1H; Indol-H⁴); 8,22 (s; 1H; Azomethin-H); 10,63 (s; 1H; -OH); 11,53 (s; 1H; Indol-NH) |

### Allgemeine Vorschrift zur Herstellung der substituierten 2-Phenylindol-3-carbaldehyd-N'-aroylhydrazone

Der entsprechend substituierte Indol-3-carbaldehyd (1 mmol) wird mit 1,2 mmol des entsprechenden Hydrazids in 20 ml 99%igem Ethanol und 4 mL Eisessig unter Rückfluss 1 Stunde erhitzt. Die noch heiße Lösung versetzt man bis zur bleibenden Trübung mit Wasser und lässt anschließend langsam abkühlen, wobei das Hydrazon auskristallisiert. Das getrocknete Produkt wird durch Umkristallisation aus Ethanol gereinigt.

### Beispiel 18

### 5-n-Butyl-2-(4-methoxyphenyl)indol-3-carbaldehyd-benzoylhydrazon

Darstellung aus 5-*n*-Butyl-2-(4-methoxyphenyl)-indol-3-carbaldehyd und Benzoesäurehydrazid, Reinigung durch Umkristallisation aus Ethanol (+4 °C).
Hellgelber Feststoff; Ausbeute: 72 %
Schmp.: 218 °C (Cu-Block)
C₂₇H₂₇N₃O₂ (425,53)

| | |
|---|---|
| IR (KBr): | ν (cm⁻¹) = 3325 (s; br; -NH); 1630 (s; -C=O) |
| ¹H-NMR (DMSO-d₆): | δ (ppm) = 0,93 (t; ³J = 7,3Hz; 3H; -CH₂-CH₂-CH₂-**CH₃**); 1,37 (sextett;³J = 7,3Hz; 2H; -CH₂-CH₂-**CH₂**-CH₃); 1,63 (quintett; ³J = 7,5Hz; 2H; -CH₂-**CH₂**-CH₂-CH₃); 2,70 (t; ³J = 7,5Hz; 2H; -**CH₂**-CH₂-CH₂-CH₃); 3,85 (s; 3H; -OMe); 7,07 (dd; ³J = 8,3Hz; ⁴J = 1,5Hz; 1H; Indol-H⁶); 7,15 und 7,92 (AA'BB'; ³J = 8,5Hz; 4H; Phenyl-H); 7,33 (d; ³J = 8,2Hz; 1H; Indol-H⁷); 7,48-7,58 (m; 5H; Benzoyl-H); 8,24 (s; 1H; Indol-H⁴); 8,71 (s; 1H; Azomethin-H); 11,50 (s; 1 H) und 11,68 (s; 1H)(Indol-NH und ―NH-CO-) |

### Beispiel 19

### 5-n-Butyl-2-(4-methoxyphenyl)-indol-3-carbaldehyd-nicotinylhydrazon

Darstellung aus 5-n-Butyl-2-(4-methoxyphenyl)-indol-3-carbaldehyd und Nicotinsäurehydrazid, Reinigung durch Umkristallisation aus Ethanol (+4 °C).
Gelber Feststoff; Ausbeute: 65 %
Zersetzung ab 125 °C
C₂₆H₂₆N₄O₂ (426,52)

| | |
|---|---|
| IR (KBr): | v (cm⁻¹) = 3292 (br, -N-H); 1661 (s; -C=O) |
| ¹H-NMR (DMSO-d₆): | δ (ppm) = 0,93 (t; ³J = 7,3Hz; 3H; -CH₂-CH₂-CH₂-**CH**₃); 1,37 (sextett;³J = 7,4Hz; 2H; -CH₂-CH₂-**CH**₂-CH₃); 1,63 (quintett; ³J = 7,5Hz; 2H; -CH₂-**CH**₂-CH₂-CH₃); 2,70 (t; ³J = 7,6Hz; 2H; **-CH₂-**CH₂-CH₂-CH₃); 3,86 (s; 3H; -OMe); 7,07 (dd; ³J = 8,3Hz; ⁴J = 1,6Hz; 1H; Indol-H⁶); 7,16 und 7,58 (AA'BB'; ³J = 8,8Hz; 4H; Phenyl-H); 7,33 (d; ³J = 8,2Hz; 1H; Indol-H⁷); 7,53 (m; 1H; Pyridin-H²); 8,23 (m; 1H; Pyridin-H³); 8,27 (m; 1H; Pyridin-H⁴); 8,69 (s; 1H; Indol-H⁴); 8,73 (m; 1H; Pyridin-H¹); 9,07 (s; 1H; Azomethin-H); 11,64 (s; 1H) und 11,70 (s; 1H)(Indol-NH und-NH-CO-) |

### Beispiel 20:

### 5-n-Butyl-2-(4-trifluormethylphenyl)-indol-3-carbaldehyd-nicotinylhydrazon

Darstellung aus 5-*n*-Butyl-2-(4'-trifluormethylphenyl)-indol-3-carbaldehyd und Nicotinsäurehydrazid, Reinigung durch Umkristallisation aus Ethanol (+4 °C).
Hellgelber Feststoff; Ausbeute: 85 %
Schmp.: 225 °C (Cu-Block)
C₂₆H₂₃F₃N₄O (464,49)

| | |
|---|---|
| IR (KBr): | v (cm⁻¹) = 3285 (m; br; -N-H); 1664 (s; -C=O) |
| ¹H-NMR (DMSO-d₆): | δ (ppm) = 0,94 (t; ³J = 7,3Hz; 3H; -CH₂-CH₂-CH₂**-CH₃**); 1,37 (sextett;³J = 7,5Hz; 2H; -CH₂-CH₂-**CH**₂-CH₃); 1,64 (quintett; ³J = 7,5Hz; 2H; -CH₂**-CH₂**-CH₂-CH₃); 2,72 (t; ³J = 7,5Hz; 2H; -**CH**₂-CH₂-CH₂-CH₃); 7,14 (dd; ³J = 8,4Hz; ⁴J = 1,3Hz; 1H; Indol-H⁶); 7,40 (d; ³J = 8,2Hz; 1H; Indol-H⁷); 7,57 (m; 1H; Pyridin-H); 7,87 und 7,96 (AA'BB'; ³J = 8,4Hz; 4H; Phenyl-H), 8,24-8,44 (m; 2H; Pyridin-H); 8,71 (s; 1H, Indol-H⁴); 8,73-8,76 (m; 1H; Pyridin-H); 9,08 (s; 1H; Azomethin-H); 11,69 (s; 1H) und 12,01 (s; 1H)(Indol-NH und-NH-CO-) |

**Tabelle 1. Erfindungsgemäße 3-Indolylmethylen-Derivate 1-30 und ihre Struktureigenschaften**

| | | | | | | |
|---|---|---|---|---|---|---|
| 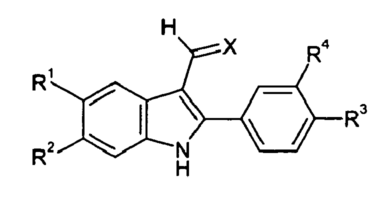 | | | | | | |

| Nr. | **Beisp.** | R¹ | R² | R³ | R⁴ | X |
|---|---|---|---|---|---|---|
| 1 | **9** | Me | H | OMe | H | C(CN)₂ |
| 2 | **10** | Me | Cl | OMe | H | C(CN)₂ |
| 3 | **11** | H | OMe | Me | H | C(CN)₂ |
| 4 | | F | H | OMe | H | C(CN)₂ |
| 5 | | Pr | H | OMe | H | C(CN)₂ |
| 6 | **12** | iPr | H | OMe | H | C(CN)₂ |
| 7 | **13** | Bu | H | OMe | H | C(CN)₂ |
| 8 | **14** | Pent | H | OMe | H | C(CN)₂ |
| 9 | **15** | Hex | H | OMe | H | C(CN)₂ |
| 10 | | H | OMe | OMe | H | C(CN)₂ |
| 11 | | H | H | OMe | H | C(CN)₂ |
| 12 | | H | OMe | H | OMe | C(CN)₂ |
| 13 | | Bu | H | CF₃ | H | C(CN)₂ |
| 14 | | Bu | H | Me | H | C(CN)₂ |
| 15 | | Bu | H | Et | H | C(CN)₂ |
| 16 | | Pent | H | CF₃ | H | C(CN)₂ |
| 17 | | Hex | H | CF₃ | H | C(CN)₂ |
| 18 | | Bu | H | OMe | H | C(CN)COOH |
| 19 | **16** | Bu | H | CF₃ | H | NMe |
| 20 | **17** | Bu | H | OMe | H | NOH |
| 21 | | H | OMe | OMe | H | NNHPh |
| 22 | **18** | Bu | H | OMe | H | 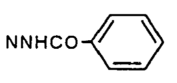 |
| 23 | | Bu | H | OMe | H | 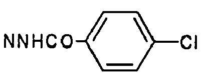 |
| 24 | **19** | Bu | H | OMe | H | 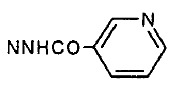 |
| 25 | **20** | Bu | H | CF₃ | H | 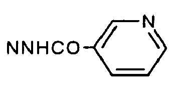 |
| 26 | | Bu | H | Et | H | 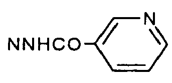 |
| 27 | | Bu | H | CF₃ | H | 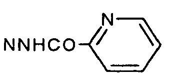 |
| 28 | | Bu | H | CF₃ | H | 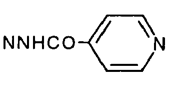 |
| 29 | | Bu | H | OMe | H | 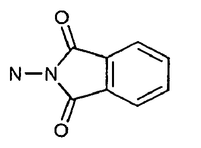 |
| 30 | | Bu | H | CF₃ | H | NNHCONH₂ |

**Tabelle 2.**

| | | | |
|---|---|---|---|
| Erfindungsgemäße 3-Indolylmethylen-Derivate der allgemeinen Formel 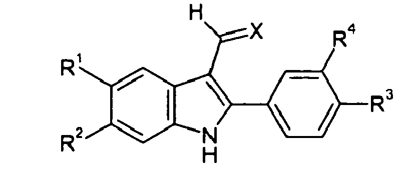 und ihre Hemmwirkung auf das Wachstum menschlicher MDA-MB-231-Brustkrebszellen | | | |

| Nr. | **Beisp.** | Verbindung | IC₅₀ [µM] |
|---|---|---|---|
| 1 | **9** | *[2-(4-Methoxyphenyl)-5-methyl-indol-3-yl]-methylenpropandinitril* | 0.28 |
| 2 | **10** | *[6-Chlor-2-(4-methoxypheizyl)-5-methyl-indol-3-yl]-methyletipropandinitril* | 0.075 |
| 3 | **11** | *[6-Methoxy-2-(4-methylphenyl)-indol-3-yl]-methylenpropandinitril* | 0.18 |
| 4 | | *[5-Fluor-2-(4-methoxyphenyl)-indol-3-yl]-methylenpropandinitril* | 0.40 |
| 5 | | *[2-(4-Methoxyphenyl)-5-n-propyl-indol-3-yl]-methylenpropandinitril* | 0.083 |
| 6 | **12** | *[6-Isopropyl-2-(4-methoxyphenyl)-indol-3-yl]-methylenpropandinitril* | 0.21 |
| 7 | **13** | *[5-n-Butyl-2-(4-methoxyphenyl-indol-3-yl]-methylenpropandinitril* | 0.013 |
| 8 | **14** | *[2-(4-Methoxyphenyl)-5-n-pentyl-indol-3-yl]-methylenpropandinitril* | 0.042 |
| 9 | **15** | *[5-n-Hexyl-2-(4-methoxyphenyl)-indol-3-yl]-methylenpropandinitril* | 0.046 |
| 10 | | *[6-Methoxy-2-(4-methoxyphenyl)-indol-3-yl]-methylenpropandinitril* | 0.26 |
| 11 | | *[2-(4-Methoxyphenyl)-indol-3-yl]-methylenpropandinitril* | 0.72 |
| 12 | | *[5-Methoxy-2-(3-methoxyphenyl)-indol-3-yl]-methylenpropandinitril* | 0.97 |
| 13 | | *{5-Butyl-2-[4-(trifluoromethyl)-phenyl]-indol-3-yl}-methylenpropanedinitril* | 0.056 |
| 14 | | *[5-Butyl-2-(4-methylphenyl)-indol-3-yl]-methylenpropandinitril* | 0.065 |
| 15 | | *[5-Butyl-2-(4-ethylphenyl)-indol-3-yl]-methylenpropanedinitril* | 0.079 |
| 16 | | *{5-Pentyl-2-[4-(trifluoromethyl)-phenyl]-indol-3-yl}-methylenepropandinitril* | 0.078 |
| 17 | | *{5-Hexyl-2-[4-(trifluoromethyl)-phenyl]-indol-3-yl}-methylenpropanedinitril* | 0.15 |
| 18 | | *3-[5-n-Butyl-2-(4-methoxyphenyl)-indol-3-yl]-2-cyanoacrylsäure* | 0.20 |
| 19 | **16** | *5-n-Butyl-2-[4-(trifluormethyl)-phenyl]-indol-3-carbaldehydmethylimin* | 0.032 |
| 20 | **17** | *5-n-Butyl-2-(4-methoxyphenyl)-indol-3-carbaldehyd-oxim* | 0.040 |
| 21 | | *6-Methoxy-2-(4-methoxyphenyl)-indol-3-carbaldehyd-phenylhydrazon* | 1.0 |
| 22 | **18** | *5-n-Butyl-2-(4-methoxyphenyl)-indol-3-carbaldehyd-benzoylhydrazon* | 0.032 |
| 23 | | *5-n-Butyl-2-(4-methoxyphenyl-)indol-3-carbaldehyd-(4-chlorbenzoyl)-hydrazon* | 0.090 |
| 24 | **19** | *5-n-Butyl-2-(4-methoxyphenyl)indol-3-carbaldehyd-nicotinylhydrazon* | 0.035 |
| 25 | **20** | *5-Butyl-2-[4-(trifluoromethyl)-phenyl]-indol-3-carbaldehyd-nicotinylhydrazone* | 0.097 |
| *26* | | *5-Butyl-2-(4-ethylphenyl)-indol-3-carbaldehyd-nicotinylhydrazon* | 0.053 |
| *27* | | *5-n-Butyl-2-[4-(trifluormethyl)-phenyl]-indol-3-carbaldehyd-picolinylhydrazon* | 0.26 |
| 28 | | *5-Butyl-2-[4-(trifluoromethyl)-phenyl]-indol-3-carbaldehydisonicotinylhydrazon* | 0.094 |
| *29* | | *N-{[5-n-Butyl-2-(4-methoxyphenyl)-indol-3-yl]-methylenamino}-phthalimid* | 0.030 |
| 30 | | *5-Butyl-2-[4-(trifluormethyl)-phenyl]-indol-3-carbaldehyd-semicarbazon* | 0.85 |

**Tabelle 3. Hemmwirkung der Beispiele 9 bis 20 auf das Wachstum von menschlichen MCF-7-Mammacarcinomzellen**

| | | | | | | | |
|---|---|---|---|---|---|---|---|
| 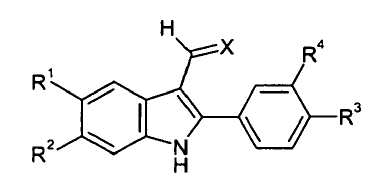 | | | | | | | |

| Nr. | **Beisp.** | R¹ | R² | R³ | R⁴ | X | IC₅₀ [µM] |
|---|---|---|---|---|---|---|---|
| 1 | **9** | Me | H | OMe | H | C(CN)₂ | 0.20 |
| 2 | **10** | Me | Cl | OMe | H | C(CN)₂ | 0.20 |
| 3 | **11** | H | OMe | Me | H | C(CN)₂ | 0.25 |
| 6 | **12** | iPr | H | OMe | H | C(CN)₂ | 0.31 |
| 7 | **13** | Bu | H | OMe | H | C(CN)₂ | 0.013 |
| 8 | **14** | Pent | H | OMe | H | C(CN)₂ | 0.076 |
| 9 | **15** | Hex | H | OMe | H | C(CN)₂ | 0.025 |
| 19 | **16** | Bu | H | CF₃ | H | NMe | 0.14 |
| 20 | **17** | Bu | H | OMe | H | NOH | 0.21 |
| 22 | **18** | Bu | H | OMe | H | 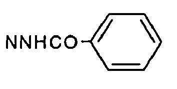 | 0.13 |
| 24 | **19** | Bu | H | OMe | H | 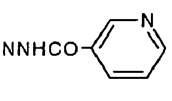 | 0.075 |
| 25 | **20** | Bu | H | CF₃ | H | 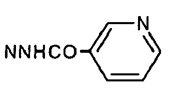 | 0.26 |

**Tabelle 4. Wirkung von (3-Indolyl)methylen-Derivaten auf den Zellzyklus von MDA-MB-231-Mammacarcinomzellen**

| | | | | | | | | |
|---|---|---|---|---|---|---|---|---|
| 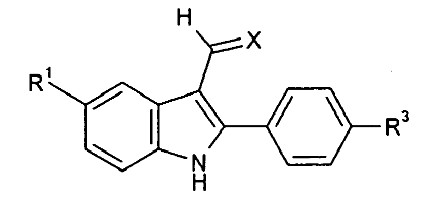 | | | | | | | | |

| Verb. (Beisp.) | R¹ | R³ | X | Konz. [µM] | Debris % | G₁/G₀ % | S % | G₂/M % |
|---|---|---|---|---|---|---|---|---|
| Kontr. | | | | | 5.6 | 51.7 | 25.7 | 17.1 |
| Vincr. | | | | 0.1 | 13.0 | 11.4 | 27.3 | 48.4 |
| 1 (**9**) | Me | OMe | C(CN)₂ | 0.5 | 7.8 | 41.0 | 19.2 | 32.0 |
| | | | | 1.0 | 16.4 | 16.9 | 21.3 | 45.4 |
| | | | | 5.0 | 6.4 | 9.8 | 26.1 | 57.7 |
| 9 (**15**) | Hex | OMe | C(CN)₂ | 0.1 | 6.1 | 46.2 | 25.3 | 22.4 |
| | | | | 0.5 | 22.6 | 7.4 | 16.1 | 53.9 |
| | | | | 1.0 | 15.9 | 7.1 | 19.7 | 57.3 |
| Vincr. | | | | 0.1 | 6.0 | 6.2 | 19.4 | 67.0 |
| 14 | Bu | Me | C(CN)₂ | 0.05 | 2.5 | 52.7 | 24.0 | 22.4 |
| | | | | 0.1 | 3.3 | 50.6 | 24.6 | 21.9 |
| | | | | 0.5 | 24.5 | 16.7 | 15.6 | 41.5 |
| | | | | 1.0 | 19.1 | 6.1 | 10.3 | 64.2 |
| Kontr. | | | | | 6.0 | 50.4 | 24.1 | 19.3 |
| Vincr. | | | | 0.1 | 8.1 | 4.5 | 9.3 | 76.0 |
| 13 | Bu | CF₃ | C(CN)₂ | 0.01 | 2.9 | 52.7 | 22.9 | 22.1 |
| | | | | 0.05 | 3.0 | 52.3 | 22.6 | 22.1 |
| | | | | 0.1 | 13.3 | 52.6 | 22.3 | 12.2 |
| | | | | 0.5 | 5.5 | 5.4 | 9.2 | 76.3 |
| 15 | Bu | Et | C(CN)₂ | 0.01 | 5.0 | 53.6 | 20.0 | 21.8 |
| | | | | 0.05 | 6.6 | 50.3 | 24.2 | 19.8 |
| | | | | 0.1 | 8.6 | 51.8 | 20.7 | 18.9 |
| | | | | 0.5 | 23.2 | 5.7 | 6.3 | 62.8 |
| Kontr | | | | | 3.5 | 42.0 | 18.0 | 35.5 |
| Vincr. | | | | 0.1 | 19.0 | 5.1 | 11.3 | 60.7 |
| 19 (**16**) | Bu | CF₃ | NMe | 0.01 | 4.4 | 43.8 | 14.0 | 36.6 |
| | | | | 0.05 | 14.6 | 38.9 | 15.3 | 30.8 |
| | | | | 0.1 | 27.1 | 5.5 | 7.2 | 59.3 |
| | | | | 0.5 | 20.5 | 6.3 | 11.1 | 59.8 |
| Kontr. | | | | | 4.2 | 50.0 | 17.6 | 29.0 |
| Vincr. | | | | 0.1 | 16.1 | 9.3 | 17.6 | 53.8 |
| 20 (**17**) | Bu | OMe | NOH | 0.05 | 7.3 | 50.0 | 13.2 | 30.0 |
| | | | | 0.1 | 12.2 | 38.6 | 14.4 | 34.9 |
| | | | | 0.5 | 9.6 | 9.0 | 14.4 | 64.6 |
| | | | | 1.0 | 13.8 | 9.6 | 21.2 | 54.8 |
| Kontr. | | | | | 4.5 | 49.6 | 22.3 | 24.4 |
| Vincr. | | | | 0.1 | 7.7 | 10.2 | 21.3 | 61.0 |
| 25 **(20)** | Bu | CF₃ | 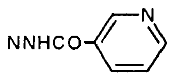 | 0.01 | 2.3 | 50.8 | 21.7 | 26.3 |
| | | | | 0.05 | 2.7 | 51.2 | 23.1 | 24.1 |
| | | | | 0.1 | 2.6 | 48.8 | 24.4 | 24.9 |
| | | | | 0.5 | 6.9 | 11.4 | 11.0 | 75.2 |
| 26 | Bu | Et | 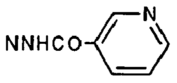 | 0.01 | 3.2 | 51.0 | 20.2 | 26.3 |
| | | | | 0.05 | 2.8 | 51.3 | 23.7 | 22.9 |
| | | | | 0.1 | 3.4 | 49.2 | 23.0 | 25.5 |
| | | | | 0.5 | 12.2 | 9.6 | 11.4 | 66.9 |
| Kontr. | | | | | 5.5 | 45.5 | 25.3 | 24.9 |
| Vincr. | | | | 0.1 | 16.6 | 8.1 | 17.2 | 54.1 |
| 28 | Bu | CF₃ | 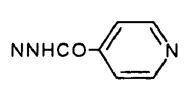 | 0.05 | 3.5 | 49.6 | 25.6 | 24.2 |
| | | | | 0.1 | 3.8 | 49.3 | 23.7 | 24.0 |
| | | | | 0.5 | 16.7 | 16.9 | 15.0 | 50.2 |

## Patentansprüche

1. 3-Indolylmethylen-Derivate mit der allgemeinen Formel 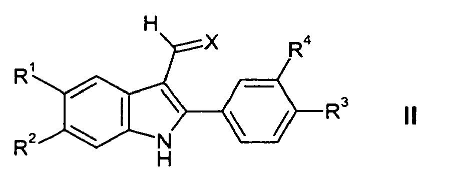 worin **R¹** und **R³** unabhängig voneinander
a) einen Wasserstoff, gerad- oder verzweigtkettige C₁- bis C₁₀-Alkyl-, -Alkenyl- oder ―Alkinreste oder
b) gerad- oder verzweigtkettige C₁- bis C₆-Alkoxyreste oder
c) Halogenatome oder
d) ganz oder teilweise fluorierte Alkylreste mit 1 ― 4 Kohlenstoffen darstellen,
**R²** Wasserstoff, einen gerad- oder verzweigtkettigen C₁- bis C₆-Alkylrest, ein Halogenatom, insbesondere Fluor oder Chlor, oder eine Alkoxygruppe mit 1 ― 6 Kohlenstoffatomen bedeutet,
**R⁴** Wasserstoff, eine Hydroxygruppe oder eine Methoxygruppe bedeutet und
**X** folgende Bedeutungen aufweist:
a) eine Methylengruppe mit einer oder zwei Carboxylgruppen oder
b) eine Methylengruppe mit einer oder zwei Carbonsäureamidgruppen, wobei die Aminogruppen entweder unsubstituiert oder mit C₁-C₆-Alkylgruppen substituiert sind, oder
c) eine Methylengruppe mit einer oder zwei Nitrilgruppen oder
d) eine Methylengruppe mit zwei unterschiedlichen unter a), b) oder c) genannten Substituenten oder
e) eine Gruppierung NR⁶, wobei R⁶ Hydroxy, Alkoxy mit einem gerad- oder verzweigtkettigen C₁- C₆-Alkylrest, ein gerad- oder verzweigtkettiger C₁- C₆-Alkylrest, oder ein Arylrest, wobei Aryl ein unsubstituierter oder substituierter, vorzugsweise ein- oder zweifach substituierter Phenylrest ist,
f) eine Gruppierung N-NR⁷R⁸, wobei R⁷ ein Wasserstoffatom oder eine gerad- oder verzeigtkettige C₁-C₄-Alkylgruppe bedeutet und R⁸ ein unsubstituierter oder substituierter, vorzugsweise ein- oder zweifach substituierter Phenylrest ist,
g) eine Gruppierung N-NR¹⁰-CO-R⁹, wobei R⁹ eine Aminogruppe, ein unsubstituierter oder substituierter, vorzugsweise ein- oder zweifach substituierter Phenylrest oder Heteroarylrest, beispielsweise 2-Thienyl, 2-Furyl, 2-, 3- oder 4-Pyridinyl, 2-, 3- oder 4-Chinolinyl, 1-, 3- oder 4-Isochinolinyl, 2-, 4- oder 5-Pyrimidyl oder 1,3,5-Trazinyl, ist und R¹⁰ ein Wasserstoffatom oder eine gerad- oder verzeigtkettige C₁-C₄-Alkylgruppe bedeutet, oder die Gruppe NR¹⁰-CO-R⁹ für ein cyclisches Amid oder Imid, vorzugsweise Phthalimid steht,
oder ein pharmakologisch verträgliches Säureadditionssalz hiervon.

2. 3-Indolylmethylen-Derivate nach Anspruch 1, wobei **X** eine Methylengruppe mit zwei Substituenten ist, die unabhängig voneinander Carboxylgruppen, Carbonsäureamidgruppen,
wobei die Aminogruppen entweder unsubstituiert oder mit C₁-C₆-Alkylgruppen substituiert sind, oder Nitrilgruppen sind.

3. 3-Indolylmethylen-Derivate nach Anspruch 1, wobei **X** NR⁶ ist, wobei R⁶ eine Hydroxygruppe oder ein C₁ ― C₆-Alkylrest ist.

4. 3-Indolylmethylen-Derivate nach Anspruch 1, wobei **X** eine Gruppierung N-NR⁷R⁸ ist,
wobei R⁷ ein Wasserstoffatom und R⁸ ein unsubstituierter oder substituierter, vorzugsweise ein- oder zweifach substituierter Phenylrest ist.

5. 3-Indolylmethylen-Derivate nach Anspruch 1, wobei X eine Gruppierung N-NR¹⁰-CO-R⁹ ist, wobei R⁹ eine Aminogruppe, ein unsubstituierter oder substituierter, vorzugsweise ein- oder zweifach substituierter Phenylrest oder Heteroarylrest, beispielsweise 2-, 3- oder 4-Pyridinyl, und R¹⁰ ein Wasserstoffatom bedeutet, oder die Gruppe NR¹⁰-CO-R⁹ für ein cyclisches aromatisches Amid oder Imid, beispielsweise Phthalimid steht.

6. 3-Indolylmethylen-Derivate nach einem der Ansprüche 1-5, wobei **R¹** einen Wasserstoff, einen gerad- oder verzweigtkettigen C₁- bis C₆-Alkylrest, eine Methoxygruppe oder ein Chlor- oder Fluoratom darstellt, **R²** Wasserstoff, eine Methoxygruppe oder ein Chlor- oder Fluoratom ist, **R³** ein Wasserstoff, ein C₁- bis C₄-Alkylrest, eine Methoxygruppe oder eine Trifluormethylgruppe ist und **R⁴** Wasserstoff, eine Hydroxy- oder eine Methoxygruppe darstellt.

7. 3-Indolylmethylen-Derivate nach einem oder mehreren der vorhergehenden Ansprüche, das aus der Gruppe ausgewählt ist, die aus
1) *[2-(4-Methoxyphenyl)-5-methyl-indol-3-yl]-methylenpropandinitril*
2) *[6-Chlor-2-(4-methoxyphenyl)-5-methyl-indol-3-yl]-methylenpropandinitril*
3) *[6-Methoxy-2-(4-methylphenyl)-indol-3-yl]-methylenpropandinitril*
4) *[5-Fluor-2-(4-methoxyphenyl)-indol-3-yl]methylenpropandinitril*
5) *[2-(4-Methoxyphenyl)-5-n-propyl-indol-3-yl]-methylenpropandinitril*
6) *[6-Isopropyl-2-(4-methoxyphenyl)-indol-3-yl]methylenpropandinitril*
7) *[5-n-Butyl-2-(4-methoxyphenyl)-indol-3-yl]-methylenpropandinitril*
8) *[2-(4-Methoxyphenyl)-5-n-pentyl-indol-3-yl]-methylenpropandinitril*
9) *[5-n-Hexyl -2-(4-methoxyphenyl)-indol-3-yl]-methylenpropandinitril*
10) *[6-Methoxy-2-(4-methoxyphenyl)-indol-3-yl]methylenpropandinitril*
11) *[2-(4-Methoxyphenyl)-indol-3-yl]-methylenpropandinitril*
12) *[5-Methoxy-2-(3-methoxyphenyl)-indol-3-yl]-methylenpropandinitril*
13) *{5-Butyl-2-[4-(trifluormethyl)-phenyl]-indol-3-yl}methylenpropandinitril*
14) *[5-Butyl-2-(4-methylphenyl)-indol-3-yl]-methylenpropandinitril*
15) *[5-Butyl-2-(4-ethylphenyl)-indol-3-yl]-methylenpropandinitril*
16) *{5-Pentyl-2-[4-(trifluormethyl-phenyl]-indol-3-yl}-methylenepropandinitril*
17) *{5-Hexyl-2-[4-(trifluormethyl)-phenyl]-indol-3-yl]-methylenpropandinitril*
18) *3-[5-n-Butyl-2-(4-methoxyphenyl-)indol-3-yl]-2-cyanoacrylsäure*
19) *5-n-Butyl-2-[4-(trifluormethyl)-phenyl]-indol-3-carbaldehyd-methylimin*
20) *5-n-Butyl-2-(4-methoxyphenyl)-indol-3-carbaldehyd-oxim*
21) *6-Methoxy-2-(4-methoxyphenyl)-indol-3-carbaldehyd-phenylhydrazon*
22) *5-n-Butyl-2-(4-methoxyphenyl)-indol-3-carbaldehyd-benzoylhydrazon*
23) *5-n-Butyl-2-(4-methoxyphenyl)-indol-3-carbaldehyd-(4-chlorbenzoyl)hydrazon*
24) *5-n-Butyl-2-(4-methoxyphenyl)-indol-3-carbaldehyd-nicotinylhydrazon*
25) *5-Butyl-2-[4-(trifluormethyl)-phenyl]-indol-3-carbaldehyd-nicotinylhydrazone*
26) *5-Butyl-2-(4-ethylphenyl)-indol-3-carbaldehyd-nicotinylhydrazon*
27) *5-n-Butyl-2-[4-(trifluormethyl)-phenyl]-indol-3-carbaldehyd-picolinylhydrazon*
28) *5-Butyl-2-[4-(trifluormethyl)-phenyl]-indol-3-carbaldehyd-isonicotinylhydrazon*
29) *N-{[5-n-Butyl-2(4-methoxyphenyl)-indol-3-yl]methylenamino}-phthalimid,* und
30) *5-Butyl-2-[4-(trifluormethyl)-phenyl]-indol-3-carbaldehyd-semicarbazon*
besteht.

8. Verfahren zur Herstellung der 3-Indolylmethylen-Derivate nach einem oder mehreren der Ansprüche 1-7 mit der allgemeinen Formel 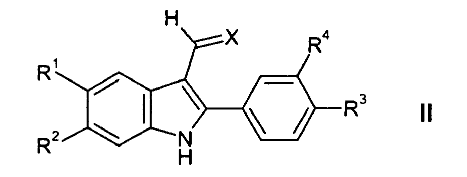 wobei **R¹, R², R³, R⁴** und **X** die in Anspruch 1 genannten Bedeutungen haben,
bei dem, ausgehend von einer Verbindung mit der allgemeinen Formel 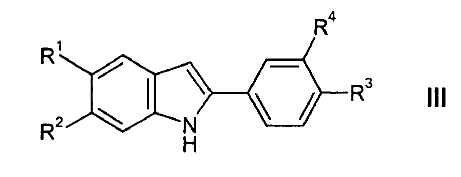 durch Einführung einer Formylgruppe in Position 3 des Indols als wichtiges Zwischenprodukt die Verbindung **IV** mit der allgemeinen Formel 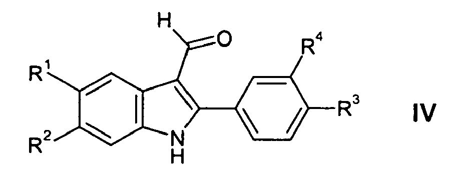 erhalten wird,
wobei die substituierten 2-Phenylindol-3-carbaldehyde der allgemeinen Formel **IV** mit C-H-aciden Verbindungen, primären Aminen, Hydroxylamin, Alkoxyaminen oder Hydrazin-Derivaten zu den 3-Indolylmethylen-Derivaten der allgemeinen Formel 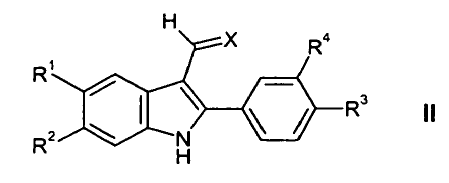 umgesetzt werden.

9. Verwendung einer Verbindung, die den Strukturteil 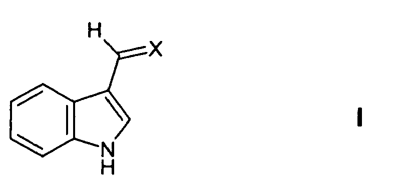 umfasst, zur Herstellung eines Arzneimittels für die cytostatische Therapie, wobei **X** die in den Ansprüchen 1 bis 5 angegebenen Bedeutungen aufweist.

10. Verwendung der 3-Indolylmethylen-Derivate nach einem der Ansprüche 1 bis 7 zur Herstellung eines Arzneimittels für die cytostatische Therapie.

11. Verwendung nach Anspruch 9 oder 10, wobei das Arzneimittel zur Behandlung von Krebs-, Viruserkrankungen, von hyperproliferativen Erkrankungen sowie Autoimmunkrankheiten vorgesehen ist.

12. Verwendung nach Anspruch 11, wobei die Krebserkrankung aus hormonunabhängigem Mammacarcinom, hormonrefraktärem Prostatacarcinom, bösartigen Tumoren von Niere und Colon, nicht-kleinzelligem Lungencarcinom und chronisch lymphatischer Leukämie ausgewählt ist.

13. Pharmazeutische Zusammensetzung, die ein 3-Indolylmethylen-Derivat nach einem der Ansprüche 1 bis 7, sowie einen pharmazeutisch verträglichen Träger umfasst.

14. Pharmazeutische Zusammensetzung nach Anspruch 13, die zusätzlich ein weiteres cytostatisches Mittel, vorzugsweise Paclitaxel, Docetaxel, Irinotecan und Gemcitabine umfasst.
